# EUROPEAN PATENT APPLICATION

(11) **EP 4 834 302 A2**
(43) Date of publication of application: **04.11.2026**
(21) Application number: 26173744.9
(22) Date of filing: 03.06.2022
(51) Int. Cl.: A61G 7/057

(54) **PRESSURE OFFLOADING DEVICE**

(30) Priority: 03.06.2021 US 202163196491 P
(62) Document number(s) of the earlier application(s) in accordance with Art. 76 EPC: 22741045.3 / 4 346 710
(71) Applicant: Recovery Force Health, LLC, Fishers, Indiana 46037 (US)
(72) Inventor: NELSON, Kevin M., Brunswick, Ohio, 44212 (US); PERSE, David, Cleveland, Ohio, 44113 (US)
(74) Representative: Müller-Boré & Partner Patentanwälte PartG mbB

(57) **Abstract**

A pressure offloading device is configured to at least partially wrap around an appendage of a patient. An example pressure offloading device includes an inner layer made of a first foam having a first density and a first indentation force deflection (IFD) rating. The pressure offloading device also includes an outer layer made of a second foam having a second density and a second IFD rating. The inner layer and the outer layer have a U-shape. Additionally, the second IFD rating is greater than the first IFD rating.

## Description

### TECHNICAL FIELD

The present disclosure generally relates to medical devices and, more specifically, a pressure offloading device.

### BACKGROUND

Healing a pressure ulcer requires protecting the wound and protecting it from pressure. Closing pressure for capillaries is typically 25-32 mmHg, but this may be compromised in pressure ulcer patients with some estimates as low as 12 mmHg, particularly in edematous patients or patients with vascular disease. Pressure alleviating devices exist but are often not used due to economic, convenience, and user interface issues. Total contact casts and removable walking casts are rarely used due to the need for specialty equipment. Other options, such as a pillow under the leg, enables the foot to shift in position, no longer providing the pressure support. Other off-loading devices are simple devices that do not keep the heel off of the bed. Additionally, while total contact casts (TCC) and removable walking casts (RWC) are effective (e.g., 89.53% of patients healing in 12 weeks for TCC and 65% for RWC, etc.), lack of facilities, the expense, messiness, and contraindications reduce their use.

### SUMMARY

Provided is a pressure offloading device configured to wrap at least partially around and support an appendage of a patient. The appendage or body portion being supported is not particularly limited. The pressure offloading device is configured to decrease or eliminate pressure of an area of the body of a person wearing the present device.

In one embodiment, the pressure offloading device is configured to support a heel (and may be referred to herein as a heel offloading device) and keep the heel off of the bed when a patient is in a supine position or positioned on their side. The pressure offloading device includes multiple foam layers. The foam layers may have different and/or varying densities, indentation force deflection values, and/or dimensions. Additionally, the foam layers are stacked to cushion and support the appendage, e.g., a heel, without fully compressing/collapsing under the weight of the patient, e.g., collapsing under the weight of a patient's leg in the case of a heel or foot offloading device. This results in a progressive foam stack that resists compression as more weight is applied. The pressure offloading device is positively attached to the patient's leg so that it does not migrate. The overall height of the pressure offloading device is adjustable in the manufacturing process to prevent contact even when placed on a soft mattress or when a heavier patient or appendage is placed on the pressure offloading device.

In some examples, the pressure offloading device is integrated into a sensor system. The sensor system includes at least one sensor associated with (e.g. attached to or incorporated within, etc.) the patient device to sense when the heel touches the bed or when the pressure offloading device is being utilized. The patient device may communicate with a visual or audible alarm when contact is made or contact of a sufficient pressure is made either instantaneously or after a set period of time. This pressure may be pre-determined based on available data or data that has been gathered and synthesized over time. Additionally, the patient device may connect via wires or wirelessly to a cell phone, monitor, or nursing station. The system sensor provides a dashboard for remote or local healthcare monitoring, such as at a nurses' station, to facilitate monitoring the state of the sensors of the connected patient device(s) (e.g., the contact status and/to the duration of contact, the pressure of the contact, and the position of the patient, etc.). The sensor system may additionally or alternatively communicate to an electronic medical records system or a cloud-based database. For example, databases may aggregate other information, such as nutrition status, blood electrolyte levels, blood pressure, and other demographic data such as wound staging, time to heal, offloading time, and compliance regarding device and system usage, to create benchmarks within and across institutions and develop best practice guidelines.

An example pressure offloading device is configured to be secured to a portion of a person's body. The pressure offloading device includes an inner layer made of a first material having a first indentation force deflection (IFD) rating, and an outer layer made of a second material having a second IFD rating. The second IFD rating is greater than the first IFD rating.

An example pressure offloading device includes multiple layers of foam affixed together having a U-shape with an inner side and an outer side. Each of the multiple layers of foam have a different indentation force deflection (IFD) rating. The pressure offloading device also includes a cover with a fastener configured to secure the pressure offloading device to an appendage within the inner side.

An example pressure offloading device is configured to be secured to a portion of a person's body. The pressure offloading device includes a first layer, a second layer, a third layer, and a fourth layer. The first layer including a single foam block having a first indentation force deflection (IFD) rating. The second layer is affixed to the first layer. The second layer includes a central portion having a second IFD rating greater than the first IFD rating and edge portions having the first IFD rating. The third layer is affixed to the second layer. The third layer has a third IFD rating that is greater than the second IFD rating. The fourth layer is affixed to the third layer. The fourth layer having a fourth IFD rating that is greater than the third IFD rating.

An example method for constructing a pressure offloading device includes placing, in a U-Shaped cavity of a molding rig, an outer layer having a first indentation force deflection (IFD) rating. The method also includes affixing, within the U-Shaped cavity of the molding rig, an outer intermediate layer onto the outer layer. The outer intermediate layer has a second IFD rating that is less than the first IFD rating. The example method includes assembling an inner intermediate layer with a first potion having a third IFD rating and a second portion having a fourth IFD rating. The third IFD rating is less than the second IFD rating and the fourth IFD rating being less than the third IFD rating. Additionally, the method includes affixing, within the U-Shaped cavity of the molding rig, the inner intermediate layer onto the outer intermediate layer. The method also includes affixing, within the U-Shaped cavity of the molding rig, an inner layer onto the inner intermediate layer, the inner layer having the fourth IFD rating.

In a first embodiment, provided is a pressure offloading device configured to be secured to a portion of a person's body, the pressure offloading device comprising: an inner layer made of a first material having a first indentation force deflection (IFD) rating; and an outer layer made of a second material having a second IFD rating; wherein the second IFD rating is greater than the first IFD rating.

In a second embodiment in accordance with the first embodiment, the first material and the second material are independently selected from a foam.

In a third embodiment in accordance with the second embodiment, the pressure offloading device further comprises a first intermediate layer between the inner layer and the outer layer.

In a fourth embodiment in accordance with the third embodiment, first intermediate layer comprises a third material selected from a foam with a third IFD rating.

In a fifth embodiment in accordance with the fourth embodiment, the third IFD rating is greater than the first IFD rating and less than the second IFD rating.

In a sixth embodiment in accordance with the fourth embodiment, the first intermediate layer comprises a first section of the third material, and a second section bordering the first section, the second section formed from a material other than the third material.

In a seventh embodiment in accordance with the sixth embodiment, the material of the first section of the intermediate layer has an IFD rating greater than the first IFD rating, and the material of the second section of the intermediate layer has an IFD rating less than the IFD rating of the first section.

In an eighth embodiment in accordance with the third embodiment, the inner layer has a first length and the first intermediate layer has a second length, and wherein the first length is longer than the second length.

In a ninth embodiment in accordance with the third embodiment, the pressure offloading device comprises a second intermediate layer between the first intermediate layer and the outer layer, the second intermediate layer made of a fourth material selected from a foam having a fourth IFD rating.

In a tenth embodiment in accordance with the ninth embodiment the first intermediate layer is made of a third material selected from a foam with a third IFD rating, and wherein the fourth IFD rating is greater than the third IFD rating and less than the second IFD rating.

In an eleventh embodiment in accordance with the ninth embodiment, wherein the inner layer includes a wing portion, wherein ends of the first and second intermediate layers are affixed to the wing portion.

In a twelfth embodiment in accordance with the first embodiment, the pressure offloading device comprises a first intermediate layer affixed to the inner layer and a second intermediate layer affixed to the first intermediate layer and the outer layer.

In a thirteenth embodiment in accordance with the first embodiment, the pressure offloading device further comprises at least one intermediate layer between the inner layer and the outer layer, wherein the inner layer defines an inner side of the pressure offloading device, wherein each of the at least one intermediate layers has a higher IFD rating the further that layer is the from inner side of the pressure offloading device, and wherein the first IFD rating is the lowest IFD rating and the second IFD rating is the highest IFD rating.

In a fourteenth embodiment in accordance with any of the first through thirteenth embodiments, the pressure offloading device comprises a cover, the cover having no seems at any point of contact between the cover and the portion of the person's body when the pressure offloading device is secured to the portion of the person's body.

In a fifteenth embodiment in accordance with any of the first through fourteenth embodiments, the inner layer and the outer layer have a U-shape.

In a sixteenth embodiment in accordance with the fifteenth embodiment, the outer layer includes a plurality of slits to facilitate the outer layer conforming to the U-shape.

In a seventeenth embodiment, provided is a pressure offloading device comprising: multiple layers of foam affixed together having a U-shape with an inner side and an outer side, each of the multiple layers of foam having a different indentation force deflection (IFD) rating; and a cover with a fastener configured to secure the pressure offloading device to an appendage within the inner side.

In an eighteenth embodiment in accordance with the seventeenth embodiment, the multiple layers of foam include: an inner layer on the inner side of the U-shape; a first intermediate layer affixed to the inner layer; a second intermediate layer affixed to the first intermediate layer; and an outer layer on the outer side of the U-shape affixed to the second intermediate layer.

In a nineteenth embodiment in accordance with the seventeenth or eighteenth embodiment, the IFD rating of the outer layer is greater than the IFD rating of the second intermediate layer, the IFD rating of the second intermediate layer is greater than the IFD rating of a center portion of the first intermediate layer, and the IFD rating of the center portion of the first intermediate layer is greater than the IFD rating of the inner layer.

In a twentieth embodiment in accordance with any of the seventeenth through ninetheenth embodiments, the IFD rating of the center portion of the first intermediate layer is at least 1.5 times greater than the IFD rating of the inner layer, the IFD rating of the second intermediate layer is at least 1.5 times greater than the IFD rating of a center portion of the first intermediate layer, and the IFD rating of the outer layer is at least 2 times greater than the IFD rating of the second intermediate layer.

In a twenty-first embodiment in accordance with the any of the seventeenth through twentieth embodiments, the cover is configured to have no seems at any point of contact between the cover and the portion of the person's body when the pressure offloading device is secured to the appendage.

In a twenty-second embodiment in accordance with any of the seventeenth through twenty-first embodiments, progression of the different IFD ratings of the multiple layers is configured to decrease pressure experienced by the appendage when the pressure offloading device is secured to the appendage.

In a twenty-third embodiment, provided is a pressure offloading device configured to be secured to a portion of a person's body, the pressure offloading device comprising: a first layer including a single foam block having a first indentation force deflection (IFD) rating; a second layer connected to the first layer, the second layer including a central portion having a second IFD rating greater than the first IFD rating and edge portions having the first IFD rating; a third layer connected to the second layer, the third layer having a third IFD rating that is greater than the second IFD rating; and a fourth layer connected to the third layer, the fourth layer having a fourth IFD rating that is greater than the third IFD rating.

In a twenty-fourth embodiment in accordance with the twenty-third embodiment, the pressure offloading device further comprises a cover, the cover configured to have no seems at any point of contact between the cover and the portion of the person's body when the pressure offloading device is secured to the portion of the person's body.

In a twenty-fifth embodiment in accordance with the twenty-third or twenty-fourth embodiment, the pressure offloading device has a U-shape when not secured to the portion of the person's body, and wherein the pressure offloading device wraps around the portion of the person's body when the pressure offloading device is secured to the portion of the person's body.

In a twenty-sixth embodiment, provided is a method comprising: placing, in a U-Shaped cavity of a molding rig, an outer layer having a first indentation force deflection (IFD) rating; affixing, within the U-Shaped cavity of the molding rig, an outer intermediate layer onto the outer layer, the outer intermediate layer having a second IFD rating that is less than the first IFD rating; assembling an inner intermediate layer with a first potion having a third IFD rating and a second portion having a fourth IFD rating, the third IFD rating being less than the second IFD rating and the fourth IFD rating being less than the third IFD rating; affixing, within the U-Shaped cavity of the molding rig, the inner intermediate layer onto the outer intermediate layer; and affixing, within the U-Shaped cavity of the molding rig, an inner layer onto the inner intermediate layer, the inner layer having the fourth IFD rating.

In a twenty-seventh embodiment, provided is a method of offloading an appendage of a patient, the method comprising: placing an appendage of a patient in the pressure offloading devices of any of the first through twenty-fifth embodiments, to offload pressure from the appendage in contact with the pressure offloading device and/or an appendage distal from the offloading device.

In a twenty-eighth embodiment in accordance with the twenty-seventh embodiment, the method comprises securing, with a strap of the pressure offloading device, to the appendage to decrease pressure experienced by the appendage.

These and other features and advantages of the present disclosure are set forth in the following specification, drawings and claims.

### BRIEF DESCRIPTION OF THE DRAWINGS

Operation of the disclosure may be better understood by reference to the following detailed description taken in connection with the following illustrations, wherein:
FIG. 1 illustrates a side view of an example pressure offloading device, in accordance with the teachings of this disclosure.
FIG. 2 illustrates a side view of another example pressure offloading device, in accordance with the teachings of this disclosure.
FIGS. 3A, 3B, 3C, and 3D illustrate perspective views of various layers of another example pressure offloading device, in accordance with the teachings of this disclosure.
FIG. 4A illustrates a side view of the example pressure offloading device of FIGS. 3A, 3B, 3C, and 3D, in accordance with the teachings of this disclosure.
FIG. 4B illustrates a cross-sectional view of the example pressure offloading device of FIGS. 3A, 3B, 3C, and 3D, in accordance with the teachings of this disclosure.
FIG. 4C is a profile view of the example pressure offloading device of FIGS. 3A, 3B, 3C, and 3D, in accordance with the teachings of this disclosure.
FIGS. 5A and 5B illustrate a variation of the example pressure offloading device of FIGS. 3A, 3B, 3C, and 3D, in accordance with the teachings of this disclosure.
FIGS. 6A, 6B, and 6C illustrate the example pressure offloading device of FIGS. 3A, 3B, 3C, and 3D in an example protective case, in accordance with the teachings of this disclosure.
FIGS. 7A, 7B, and 7C illustrate an example cylindrical pressure offloading device, in accordance with the teachings of this disclosure.
FIG. 8 illustrates an example system to monitor pressure offloading devices and/or wound dressings and collect related telemetry data, in accordance with the teachings of this disclosure.

### DESCRIPTION

As described herein, a pressure offloading device is made of a compressible material that is positioned to be secured to a portion of a person's body (e.g., a patient) to elevate or offload a portion of the person's body. In one embodiment, the pressure offloading device comprises multiple foam layers. The pressure offloading device can be secured to a portion of a person's body to elevate and offload pressure (e.g., decrease or eliminate pressure) of the portion to which the device is secured. Alternatively, the device can be used to elevate and/or offload pressure of a portion of the body different from the portion of the body to which the device is secured. For example, the device can be positioned on the lower leg along the calf to function as a heel offloading device to keep a patient's foot from contacting a support surface such as a bed. The pressure offloading device may be used in conjunction with a dressing (e.g., including a substrate with adhesive, a hydrogel adhesive border and/or an absorbent pad, etc.). The effectiveness of the offloading does not change when the patient moves or the portion of the body to which it is attached changes position.

In one embodiment, the pressure offloading device includes a series of stacked foam layers that provide a progression of cushioning between an appendage being supported (e.g., the leg) and the support surface. Layer(s) closest to the appendage may be made of a relatively conforming or higher cushioning foam layer having a relatively low indentation force deflection rating. As layers extend away from the appendage, the layers may be made of more resilient foams that have higher indentation force deflection ratings. In some examples, a layer may be made of foam blocks of different indentation force deflection rating. In some such examples, the portion of a layer in line with the direct weight of the appendage when the patient is supine (e.g., the middle portion of the layer) may have higher indentation force deflection ratings than the other portions of the layer. In some such examples, the middle portion of the layer may provide greater support for the appendage and the side portions may conform more to the shape of the appendage. The outer layer(s) of the pressure offloading device may be made of a foam rigid enough to provide structure to the pressure offloading device. In some examples, the outer most layer may be made of a low indentation force deflection foam to provide protection to other body parts that may come in contact with the pressure offloading device. In some examples, the outer layer may be compatible with fixed or temporary materials or apparatuses that limit, alter, or improve the overall mobility of the pressure offloading device (e.g., wedges secured by Velcro to the sides of the pressure offloading device to limit rotation of the patient's leg). An external apparatus may also be used for securing the pressure offloading device to the patient or another device (e.g. a harness secured to a wheelchair or around the patient's waist which connects to the outer layer of the pressure offloading device to maintain. In some examples, multiple pressure offloading devices, of the same or different size, may be able to integrate or connect to one another.

The series of stacked foam layers of the pressure offloading device have one more differing properties such, for example, density, and firmness, etc. In one embodiment, the certain layers within the device are provided such that one or more layers differ with respect to the material's resistance to local surface deformation. This resistance to local surface deformation can be evaluated using any suitable test or method for evaluating such properties including, but not limited to indentation, hardness, etc. In one embodiment, indentation is measured by an indentation force deflection (IFD) rating (sometimes referred to as "indentation load deflection" or "ILD")), etc. As used herein, (i) the densities are given as measured by ASTM D 3574 - Test A as specified by the American Society for Testing and Materials, and (ii) the IFD rating is given as measured by ASTM D 3574 - Test B₁ as specified by the American Society for Testing and Materials. Other measurement methodologies may be used for the properties of the foam material as described below. The IFD rating and the density are not correlated and, as discussed below, foam with different combinations of density and IFD rating may be used. The foam composition of each layer may be selected with differing properties to facilitate, for example, weight distribution, a gradient or progression of pressure relief, a target cushion area, and/or a durability requirement (e.g., outer layers may need to be more durable than inner layers), etc. For example, a pressure offloading device may include (a) a first layer of a viscoelastic foam having a relatively low density and/or IFD rating that conforms to the leg of the patient, (b) a second layer that includes series of blocks having a higher density and/or IFD rating than that the first layer, optionally with air gaps between adjacent blocks, (c) a third layer that is continuous and has a higher density or IFD rating than the second layer, and/or (d) a fourth layer of blocks spaced adjacent to each other, the density of which is between or equal to that of the second and third layers. In some examples, one or more layers may include one or more foam blocks that have different densities or IFD rating than the adjacent block. In the example described above, the blocks of the second layer may have different densities (sometimes referred to as a "composite layer" or a "multiple density layer"). For example, a middle block may have a higher density and/or IFD rating than that the first layer, while outer blocks on either side of the middle block may have a density and/or IFD rating that is the same as the density of the first layer. A higher IFD rated middle block (e.g., the block that is placed directly underneath the patient's leg) would contribute to the gradient or progression of pressure relief where pressure is mostly commonly observed because patients are most often in the supine position. The lower IFD rated blocks of a layer promote a more conforming and form-fitting fit with the sides of a patient's leg. Additional layers may be added to the pressure offloading device as needed. Additionally, the pattern of densities and IFD ratings of the layers may facilitate reducing friction at transition zones by having less deflection (e.g. IFD rating) of layers towards those points of contact. While the layers herein are described below as being comprised of one or more solid foam blocks, the layers may be comprised of other materials that provide similar density and deflection qualities as described herein. For example, one or more of the foam layers may made of shredded foam, gel, or air bladder.

In some examples, adjacent layers may be affixed to each other along their length. In some examples, the length-wise edges of the layers may meets at a flush edge. Alternatively, in some examples, the length-wise edges of the layers may not be flush. In some examples, layers comprising blocks may only affix one or more, but not all, blocks to the neighboring layer (e.g., the blocks in the middle of the device may be affixed to the next layer and the outer blocks are left free, etc.) to enable the ends of the layers to slide amongst each other when the device is flexed to, for example, improve the fit around the leg and prevent slipping of the pressure offloading device when it is being worn.

The pressure offloading device may be a standalone device or may be part of a system that includes a wound dressing. The layers may vary in length or material content so that the device bends into position without unduly forcing the legs apart. For instance, there may be more material in the central region and less materials in the regions of the device near the ends. There may also be examples of the device with an outermost layer of low IFD material for applications where the device could be making contact with another part of the patient's body.

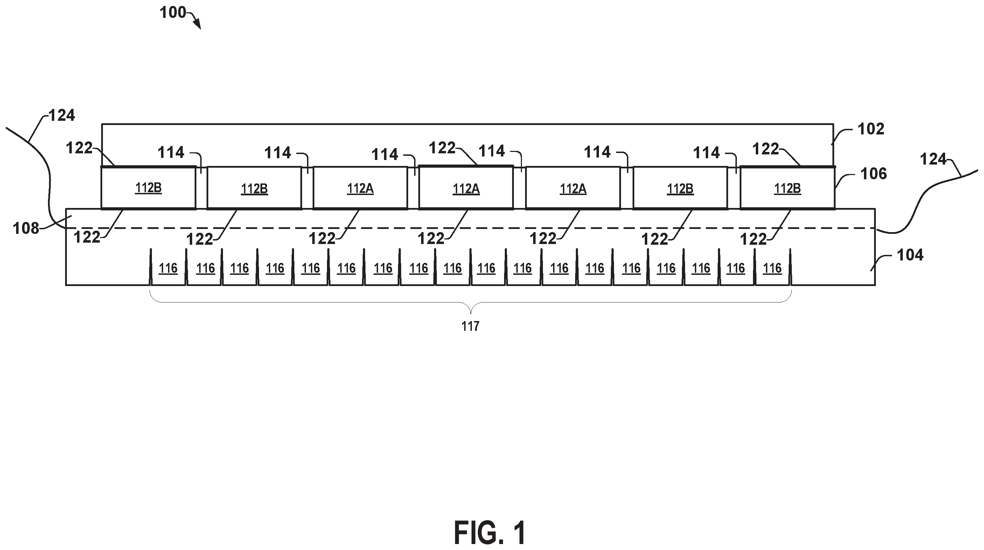

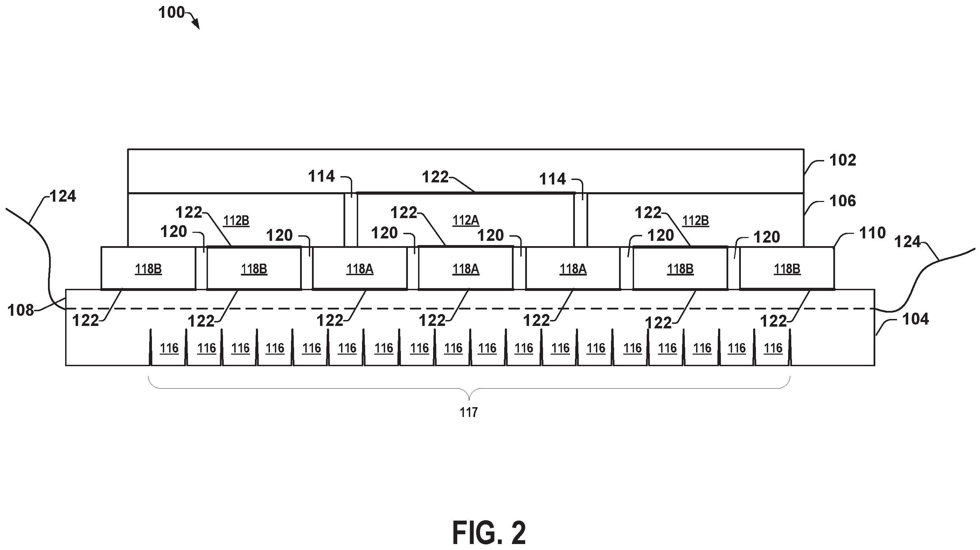

As illustrated in FIGS. 1 and 2, a device 100 (sometimes referred to as "pressure offloading device") includes a series of stacked foam layers. The pressure offloading device 100 has an inner layer 102 (e.g., a layer that contacts the patient), an outer layer 104 (e.g., a layer that contacts the support surface), and one or more intermediate layers 106, 108, and 110. The inner layer 102 is made of a viscoelastic foam that, when in use, conforms to the leg of the patient. The example inner intermediate layer 106 is made of a series of blocks 112A and 112B (collectively "blocks 112") with air gaps 114 between them. The blocks 112 of the inner intermediate layer 106 have a higher density and/or indentation force deflection (IFD) value than that of the foam of the inner layer 102. In some examples, the inner intermediate layer 106 is a composite layer. The inner intermediate layer 106 may have some portions that have a higher density and/or IFD value than other portions of the layer. The inner layer 102 and the inner intermediate layer 106 may together provide a progression of cushioning to support the leg of the patient. An example outer intermediate layer 108 is made of a continuous foam block. The foam of the outer intermediate layer 108 has a higher density and/or IFD than the inner intermediate layer 106. An example outer layer 104 is made of or define foam blocks 116 spaced adjacent to each other. The foam blocks 116 of the outer layer 104 have a density and/or IFD that is between or equal to either the density of the foam of the inner intermediate layer 106 or the foam of the outer intermediate layer 108. The outer layer 104 and outer intermediate layer 108 may provide structure to the pressure offloading device 100, and not provide to the progression of cushioning of the pressure offloading device 100.

In some examples, the layers 104 and 108 may be collectively referred to as the "off-loading zone" of the pressure offloading device 100. The remaining layers, e.g., layers 102 and 106 in FIG.1 or layers 102, 106, and 110 in FIG. 2 may be referred to as the "cushion zone."

As shown in FIG. 2, in some examples, the pressure offloading device 100 includes a middle intermediate layer 110 between the inner intermediate layer 106 and the outer intermediate layer 108. The middle intermediate layer 110 may be a layer with multiple IFD values where some portions of the layer have a higher density and/or IFD value than the other portion of the layer. The middle intermediate layer 110 is made of a series of foam blocks 118A and 118B (collectively "blocks 118") with air gaps 120. The foam of the middle intermediate layer 110 may be have a higher IFD than the foam of the inner intermediate layer 106 and lower IFD than the foam of the outer intermediate layer 108. In some examples, the middle intermediate layer 110 may be a composite layer where some blocks 118A have a higher IFD than other blocks 118B.

In some examples, the inner layer 102 and the inner intermediate layer 106 are affixed to each other along their length. Alternatively, in some examples, the one or more inner blocks 112A of the inner intermediate layer 106 are affixed to the inner layer 102 and the outer blocks 112B of the inner intermediate layer 106 are left free to enable the ends of the inner layer 102 and the inner intermediate layer 106 to slide amongst each other when the pressure offloading device 100 is flexed to, for example, improve the fit around the leg and prevent slipping of the pressure offloading device 100 when it is being worn. For illustrative purposes, connection points 122 are shown in FIGS. 1 and 2 to illustrate where the layers may be connected. Interfaces that are not illustrated with the connection points 122 may be unconnected and left free-floating relative each other. Alternatively, in some examples, the layer may be fully attached to each other in a manner that does not facilitate certain sections sliding relative to other sections.

In the illustrated example of FIGs. 1 and 2, the outer intermediate layer 108 is thinner than the other layers so that, for example, it maintains its flexibility even as it is more rigid than the other layers. The outer intermediate layer 108 may provide a structure to the overall device. The outer layer 104, which is thicker or of the same thickness to that of the outer intermediate layer 108, provides flexibility by being made of blocks 116. The blocks of the of the outer layer 104 may be individual blocks 116 that are completely separated from and spaced apart from one another, or by having back-scores 117 (as shown in FIGS. 1, 2, and 3A, 3B, 3C, and 3D). The back-scores are slits that do not extend all the way through to the outer intermediate layer 108. The width of the blocks 116 can be selected as desired. In one embodiment, the blocks 116 may be one half to two inches in width. In some examples, the blocks 116 of the outer layer 104 are one inch in width.

At least one strap 124 is attached to one end of the pressure offloading device 100. Another strap 124 may be attached to the opposite side of the pressure offloading device 100 so that the pressure offloading device 100 wraps up and around the leg. The strap(s) 124 may be secured with hook and loop, (a) buckle(s) or other such device(s). In some examples, the strap 124 is inserted into a loop and pulled back upon itself, having hook and loop materials that can be attached to each other. The strap can be positioned or affixed to any location on the pressure offloading device 100 as desired to provide for ease of use of the strap while providing a sufficient position of strength to ensure the integrity of the pressure offloading device 100 (e.g., so as not to promote adjacent layers from pulling away from one another in use or application of the pressure offloading device 100, etc.). In one embodiment, the strap may be affixed, for example, between layers 104 and 108. In examples where multiple straps are incorporated, they may affix to a single receiving material or multiple receiving materials to allow for additional conformance. In some examples, there are multiple straps 124 to allow the device to optimally contour the user's leg, as the leg itself typically varies in diameter along position of the leg. It will be appreciated that the straps do not have to be provided as part of the layered structure of the offloading device. As noted in other embodiments described herein, the pressure offloading device can include a cover layer surrounding the layered structure, and a strap or connective piece can be provided as part of the cover layer.

Within a given layer of the cushion zone, the blocks within that layer can be the same or different from one another in terms of their widths and/or the foam properties of a given block. For example, as illustrated in FIG. 1, the blocks 112 are all of the same width and formed from the same foam material. In another example, at least one block 112 has one or more properties that differ from one or more properties of another one of the blocks 112 within that layer. For example, a foam block near the center of the pressure offloading device 100 may have a density and/or IFD value that is higher than that of blocks position toward the ends of the pressure offloading device 100. In some examples, the blocks 112A near the center of the pressure offloading device 100 have a density and/or an IFD that is higher than the density and/or IFD of the remaining blocks 112B.For example, as illustrated in FIG.2, the inner layer 102 has an IFD less than the blocks 112 of the inner intermediate layer 106, and the blocks 112 of inner intermediate layer 106 have and IFD less than blocks 118 of middle intermediate layer 110.

In another example of the pressure offloading device 100 illustrated in FIG. 2, the center block 112A of the inner intermediate layer 106 has an IFD greater than the IFD of the foam of inner layer 102, the two end blocks 112B of inner intermediate layer 106 have an IFD less than the center block 112A, and the blocks 118 of the middle intermediate layer 110 each have an IFD greater than that of each of the blocks 112. In some examples, the two end blocks 112B of the inner intermediate layer 106 are formed from the same foam material as the foam of the inner layer 102. In some examples, the blocks 118A and 118B can be the same or different from one another and can have the same or different densities or IFD values. In some examples, the blocks 118A have a density or IFD greater than the IFD of blocks 118B (and the blocks 118B can have a density and/or IFD that is greater than, the same as, or less than the density and/or IFD of blocks 112B). These are merely examples, and the properties of the foam in the respective layers can be selected as desired to provide a desired level of support and/or cushioning.

The pressure offloading device 100 may be of any length and width, and thickness to accommodate a variety of patients or depending on the appendage that the device is intended to support. For example, a small device may have a 10x6 inch footprint, while an extra-large device may have a 36x12 inch footprint, where the long axis is perpendicular to the axis of the leg. The inner layer 102 and the inner intermediate layer 106 may be shorter in length than the outer intermediate layer 108 and the outer layer 104.

Additionally, the thickness of the respective layers can be the same or different from one another. In one embodiment, for example, layers 104 and 108 can have the same thickness. In another embodiment, layer 104 has a thickness greater than the thickness of layer 108. Similarly, the thickness of the respective layers in the cushioning zone can be same as one another or can differ from one another. The difference in the thickness of the layers can be selected as desired provided the thickness of the layers allows for the device to be wrapped or bent to at least partially surround or encompass the appendage to be supported.

The air gap 114 between blocks 112 in the inner intermediate layer 106 are spaced to allow the blocks 112 to fill in the gap when the pressure offloading device 100 is wrapped around the leg. The blocks 116 on the outer layer 104 are configured to improve conformability and reduce resistance when forming the device around the leg, being short across the length of the pressure offloading device. For example, the blocks 116 may be 1x6 inches.

Additionally, while the offloading zone formed by layers 104 and 108 is desirable formed from a high density and/or high IFD material, it will be appreciated that the device could optionally include a low density and/or low IFD material underlying the layer 104. This may be particularly desirable in a situation where the device, in operation, will be positioned such that the outer portion of the device, when folded, would contact another part of the patient (other than the appendage being supported). In this case, the softer, lower density and/or lower IFD material may avoid or reduce irritation to another part of the patient.

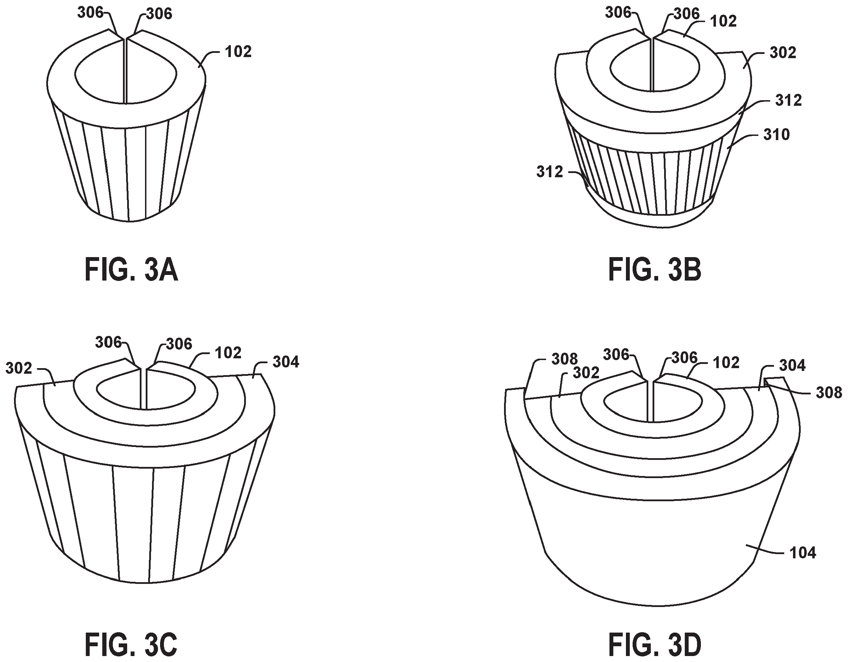

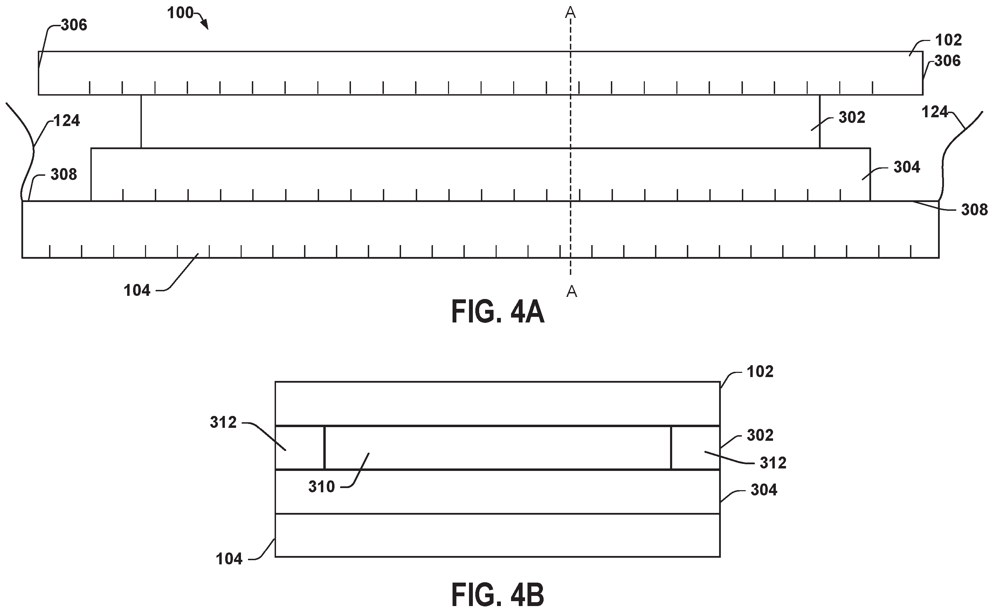

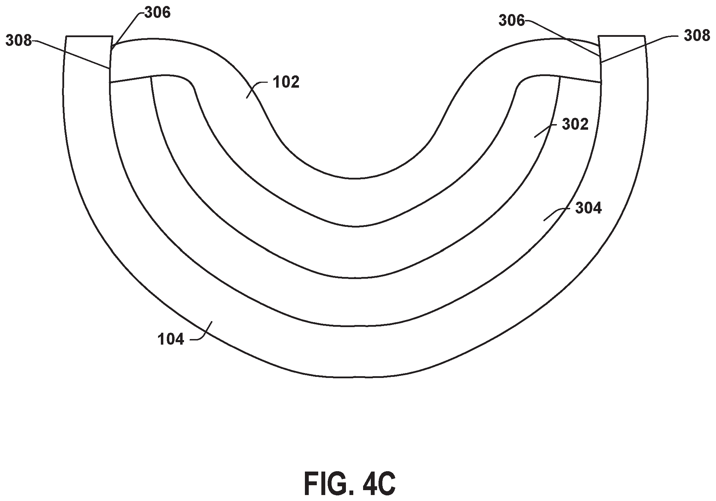

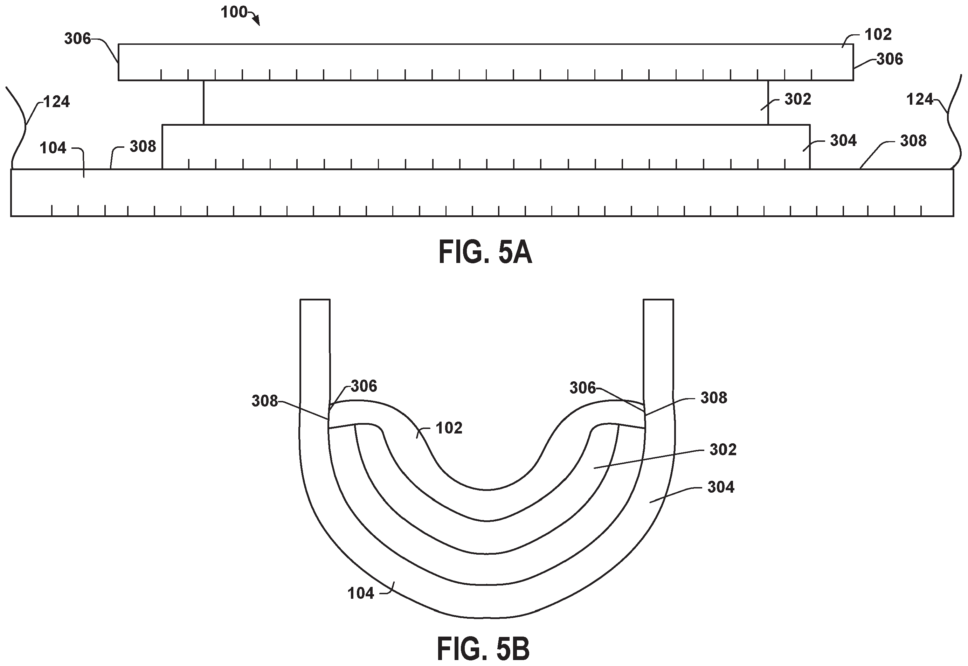

FIGS. 3A, 3B, 3C, 3D, 4A, 4B, 4C, 5, and 6 illustrate an example of the pressure offloading device 100 with one or more layers that incorporate foam with different densities and/or IFDs. FIGS. 3A, 3B, 3C, and 3D illustrate examples with one or more layers of the pressure offloading device 100 removed for illustrative purposes. FIG. 4A illustrates a side view of the pressure offloading device 100. FIG. 4B illustrates the A-A cross-section of the pressure offloading device 100 illustrated in FIG. 4A. FIG. 4C illustrates a profile view of the pressure offloading device 100 with the layers molded into a U-shape (sometimes referred to as a "C-shape"). In the illustrated examples, the pressure offloading device 100 includes an inner layer 102, an outer layer 104, and one or more intermediate layers 302 and 304. FIGS. 5A and 5B illustrate the pressure offloading device 100 where the outer layer 104 is elongated to provide extra support to an appendage. In general, to reduce friction at transition zones, the IFD rating of the layers is lower the closer it is to the point of contact to the patient. The inner layer 102 is made of a low density and low IFD viscoelastic foam (e.g., a density of 6 lb/ft³ and an IFD rating of 18 pound-force (lbf)) that conforms to the leg of the patient. The inner layer 102 may be longer than the intermediate layers 302 and 304 such that, when assembled, ends 306 may be affixed to a corresponding inner edge 308 of the outer layer 104 as illustrated, for example, in FIG. 4C. This elongated portion of the inner layer 102 is sometimes referred to as the "wing" of the inner layer 102. Because the wing of the inner layer 102 has the lower IFD rating of the inner layer 102, it shields the point of contact on the patient from the layers with higher IFD ratings (e.g., the outer layer 104 and the outer intermediate layer 304, etc.). Additionally, as illustrated in FIG. 4C, when assembled but not in use (e.g., affixed around an appendage of a patient, etc.), the pressure offloading device 100 may have a generally U-shape.

The outer layer 104 is made of a viscoelastic foam with a relatively high density and a relatively high IFD) (e.g., a density of 6.5 lb/ft³ and an IFD rating of 120 lbf)). The viscoelastic foam of the outer layer 104 may have a higher density and/or IFD value than the viscoelastic foam of the inner layer 102.

The inner intermediate layer 302 is a multiple density and/or IFD value layer that includes at least two types of viscoelastic foam that have different densities and/or different IFD values. In the illustrated example, the inner intermediate layer 302 has a core portion 310 and edge portions 312. As best illustrated in FIGS. 3B and 4B, the edge portions 312 are adjacent to two sides of the core portion 310. The viscoelastic foam of the core portion 310 has a higher density and/or IFD value than the viscoelastic foam of the inner layer 102 and the viscoelastic foam of the edge portions 312 (e.g., a density of 6 lb/ft³ and an IFD rating of 32 lbf). In the illustrated example of FIG. 3B, the core 310 runs the entire length of the inner intermediate layer 302. However, alternatively in some examples, the core 310 may run less than the entire length (e.g., take up the middle third of the inner intermediate layer 302, taking up a centered 50 percent of the inner intermediate layer 302, etc.). In some examples, the edge portions 312 may be made of the same viscoelastic foam and/or have the same properties as the inner layer 102 (e.g., a density of 6.5 lb/ft³ and an IFD rating of 18 lbf). Alternatively, in some examples, the foam of the edge portions 312 may have a density and/or IFD value between (a) the density and/or IFD value of the core portion 310, and (b) the density and/or IFD value of the outer layer 104.

In the illustrated example, the outer intermediate layer 304 is made of a foam a relatively low density and a relatively high IFD (e.g., a density of 4.75 lb/ft³ and an IFD rating of 60 lbf). In some examples, the foam of the outer intermediate layer 304 has a density and/or IFD that is greater than the density and/or IFD value of the foam of the inner layer 102 and the density and/or IFD value of the foam of the core portion 310. In some examples, the outer intermediate layer 304 may also be a multiple density and/or IFD value layer that includes at least two types of viscoelastic foam that have different densities and/or different IFD values. For example, the outer intermediate layer 304 may have a core portion made of a relatively high density and/or high IFD value foam and edge portions made of a less dense and/or lower IFD value foam. To promote flexibility, the inner layer 102, the outer layer 104, and the core 310 of the inner intermediate layer 302 may optionally be scored. Additionally, in some examples, the outer intermediate layer 304 may be scored. Example densities and IFD ratings of the layers 102, 104, 302, and 304 are described on Table 1 below.

**Table 1**

| Layer | Density (lb/ft³) | IFD Rating (lbf) |
|---|---|---|
| Inner | 6 ± 0.3 | 18 ± 3 |
| Inner Intermediate (Central Portion) | 6 ± 0.3 | 32 ± 3 |
| Outer Intermediate | 4.75 ± 0.25 | 60 ± 6 |
| Outer | 6.5 ± 0.25 | 120 ± 15 |

The densities and IFD ratings provided on Table 1 are example values. In some examples, the inner layer 102 may have an IFD rating from 2 to70 lbf, the central portion 310 of the inner intermediate layer 302 may have an IFD rating form 2 to70 lbf, the outer intermediate layer 304 may have an IFD rating of 18 to 120 lbf, and the outer layer 104 may have an IFD rating of 18-150 lbf, where each layer has a higher IFD rating as it is further from the inner layer 102. The IFD rating selected within the range described above for the inner layer 102 (and thus the subsequent layers 104, 302, and 304, etc.) may be based on (i) the portion of the body of the patient to be cushioned (e.g., heavier and/or larger appendages may start with a higher IFD rating for the inner layer 102), and/or (ii) the weight of the intended patient. For example, pressure offloading devices 100 intended to pediatric and/or geriatric patients may have an inner layer 102 with a lower IFD rating (e.g., an IFD rating of 2 lbf), pressure offloading devices 100 intended for adults may have an inner layer 102 with a medium IFD rating (e.g., an IFD rating of 18 lbf), and pressure offloading devices 100 intended for bariatric patients may have an inner layer 102 with a relatively higher IFD ration (e.g., an IFD rating of 60 lbf), etc. While FIGS. 4A, 4B, and 4C illustrate the pressure offloading device 100 having four layers, the pressure offloading device 100 may have more than four layers, where the IFD rating of each layers gets higher the farther from the inner layer 102 it is. The differences in IFD ratings between layers should be relatively gradual such that a foam layer with a low IFD rating is not adjacent to a foam layer with a high IFD rating. For example, an additional layer between the inner intermediate layer 302 and the outer intermediate layer 304 may have an IFD rating of 44. As another example, an additional layer between the outer intermediate layer 304 and the outer layer 104 may have an IFD rating of 85. In some examples, the IFD rating of a layer is based on the IFD rating of the adjacent layer that is closer to the inner layer 102. For example, the IFD rating of the outer layer 104 may be based on the IFD rating of the outer intermediate layer 304. In some examples, the IFD rating of a layer may be 1.5 to 2.5 times the IFD rating of the adjacent layer that is closer to the inner layer 102. For example, (i) the IFD rating of the inner intermediate layer 302 may be 1.5 times the IFD rating of the inner layer 102, (ii) the IFD rating of the outer intermediate layer 304 may be 1.5 times the IFD rating of the inner intermediate layer 302, and (iii) the IFD rating of the outer layer 104 may be 2.5 times the IFD rating of the outer intermediate layer 304. In some examples, when creating the pressure offloading device 100 for a patient or a patient group with similar characteristics (e.g., age, weight, height, etc.), a suitable IFD rating for the patient or patient group may be selected for the inner layer 102, and the IFD ratings of the subsequent layers may be selected based on the multipliers describe above.

In the illustrated example of FIGS. 5A and 5B, the outer layer 104 is elongated such that, when the layers 102, 104, 302, and 304 are affixed together in the U-shape form, the outer layer 104 extends such that, when the pressure offloading device 100 is attached to an appendage, portions of the outer layer 104 at least partially wrap around the appendage to provide additional cushioning effect to the appendage. In some examples, the elongated portion of the outer layer 104 may be incorporated into the straps 124 and/or, when inserted into the protective cover 600, be incorporated into the portion of the protective cover 600 that forms the strap(s) 602.

It will be appreciated that the layer 302 may include an edge portion 312 along each of the longitudinal edges of the device, and the core portion 310 disposed between the two edges. In another embodiment, the edge portions 312 may form a frame (with an edge adjacent each of the edges including the distal edges) with the core portion 310 disposed interior to the edges.

FIGS. 3A-3D may also be considered an embodiment of a method for forming the device. In one embodiment, the inner layer (e.g., inner layer 102) may be formed into curved shape around a mold piece or mandrel (not shown), and the subsequent layers may be built up around the inner layer. Alternatively, in some embodiments, to manufacture the pressure offloading device 100, the layers 102, 104, 302, and 304 are placed in a U-shaped rig (not shown). In such embodiments, the pressure offloading device 100 is constructed in a bottom-up production where the outer layer (e.g., outer layer 104) is the first layer formed into curved shape inside the U-shaped rig, and the subsequent layers (e.g., layers 304, 302, and 102) are built up upon the outer layer 104 in reverse order. Between affixing layers 304, 302, and 102, pressure and/or heat may be applied to the layers in the rig. Additionally, between adding a new layer, a dwell time may be waited.

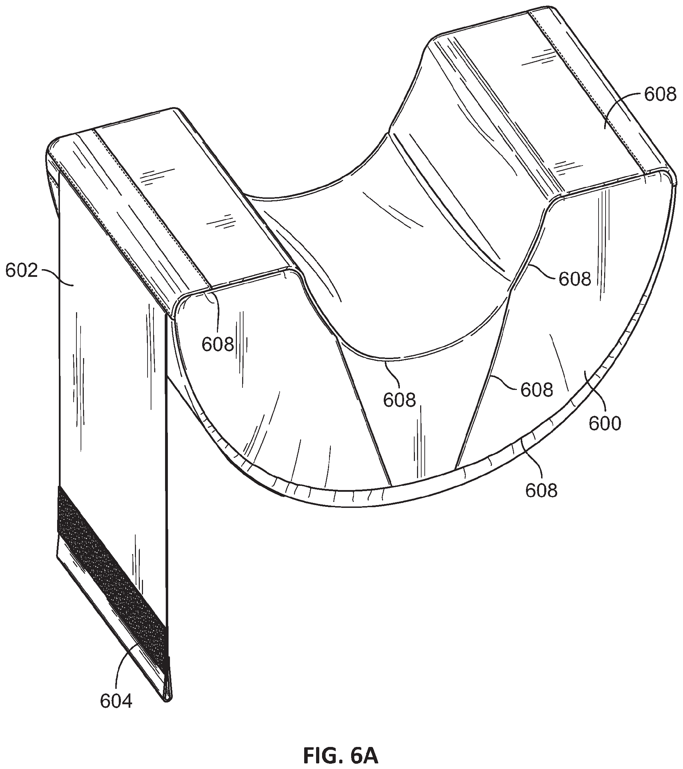

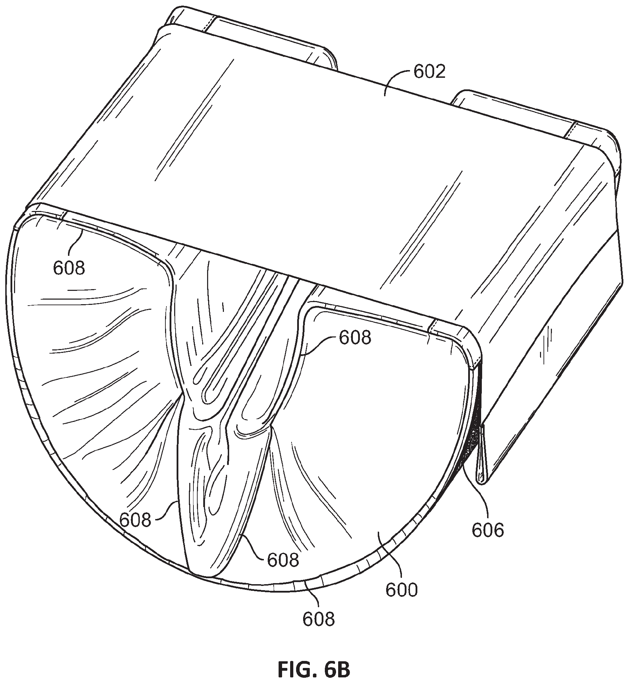

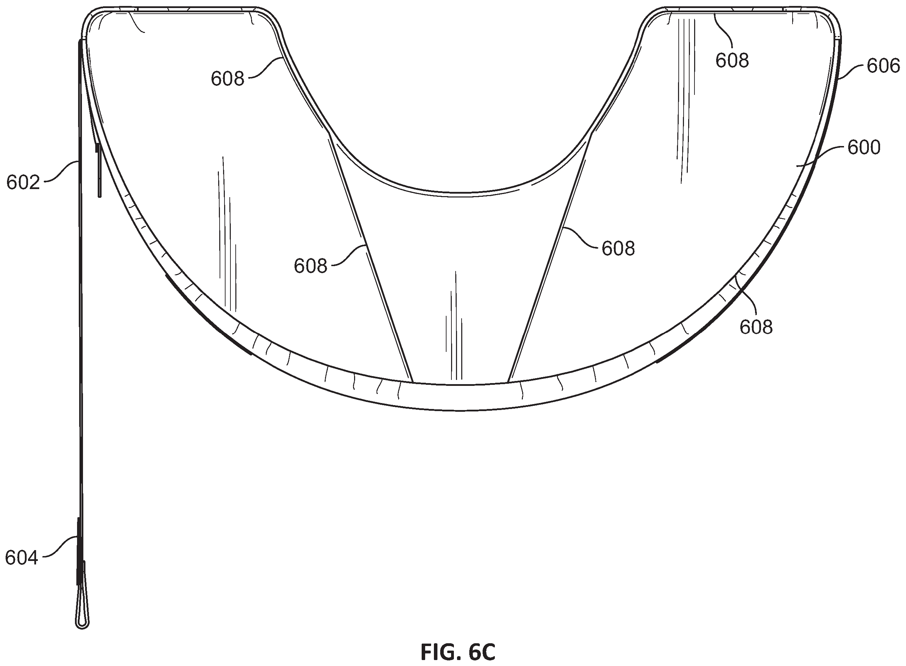

As shown in FIGS. 1, 2, 4A and 4B, and 5A, the device is shown in a flat, opened state. To support an appendage, the appendage would be positioned in a location generally central to the ends of the device, and the opposing ends of the device would be wrapped upwardly toward one another and secured using an attachment piece such as, for example, the straps 124. In one embodiment, the pressure offloading device 100 may be pre-shaped into an essentially U-shape to enable easy placement of the pressure offloading device 100 without undue strain on the user, for example, as shown in FIGS. 4C, 6A, 6B, and 6C. This shape may be achieved by forming certain layers of the device in a flat position while other layers may be formed in partially flexed position in order to achieve the desired U-shape.

In some examples, the pressure offloading device 100 includes a fabric covering that is breathable and configured to reduces friction at transition zones (e.g., the points of contact between the patient and the pressure offloading device 100). In some examples, the covering is a seamless tube along its length. In some examples, seams of the covering are oriented so that the seams do not make contact with the patient so as to not create irritation to the wearer's skin (e.g., are oriented to the bottom of the device or away from the wearer's skin). The pressure offloading device 100 may be covered in a material amenable to cleaning and may be impermeable to water while being breathable to protect the layers from being contaminated. The pressure offloading device 100 may include a removable fabric cover that can be washed. The fabric of the cover may be made of any sort of material, it may be breathable, a natural material or synthetic. The fabric of the cover may be a soft, low friction material. In some examples, the cover is made of sheepskin. In one embodiment, the cover is constructed in a shape that substantially mirrors the shape of the pressure offloading device. For example, the cover can be constructed in a u-shape conforming to the shape of the device. Having a cover that is pre-formed in a shape corresponding to the shape of the device can prevent deformation of the underlying construction of the layered pressure offloading device. For example, in a device comprising a plurality of foam layers, a "flat" cover may pull on the layered construction causing it to lose its shape. The present covers may contribute to the maintained resilience and retention of the layered construction over time.

FIGS. 6A, 6B, and 6C illustrates the pressure offloading device 100 inside a protective cover 600 that includes a strap 602 with a first fastening end 604. The cover 600 is made of an elastic material to reduce the generation of wrinkles when the pressure offloading device 100 is fastened to a patient. The strap 602 with the fastening end 604 is configured to fasten together with a second fastening end 606. The fastener ends 604 and 606 may be portions of any appropriate fastening technique, such as Velcro (e.g., where the first fastening end 604 is the hook side and the second fastening end 606 is the loops side), an adjustable quick release buckle (e.g., where the first fastening end 604 is the male buckle and the second fastening end 606 is the female buckle), a buckle and clasp (e.g., where the first fastening end 604 is the buckle and the second fastening end 606 is the clasp), etc. Additionally or alternatively, the protective cover 600 may define slots through which the straps 124 affixed to the outer layer 104 may be pulled. To reduce friction at points of contact between the patient and the protective cover 600, the protective cover 600 is seamless in all areas that, in operation, come in contact with the patient. That is, seams 608 are located on the protective cover 600 such that the seams 608 will not come in contact with the patient when in use. In some examples, the cover 600 may incorporate or include as a separate inner lining a layer or envelope of foam with low IFD rating that, for example, protects and/or cushions other parts of the patient's body that may come in contact with the pressure offloading device 100. In the illustrated example, the strap 602 is configured to directly attach to the protective cover 600. In some examples, the strap 602 may be configured to (i) attach, via the first fastening end 604, to a stabilizing device (e.g., a device that limits movement of the patient and/or otherwise supports the patient while using the pressure offloading device 100) while a strap of the stabilizing device in attaches to the second fastening end 606, or (ii) extend around the stabilizing device to and attach to the second fastening end 606 while securing the stabilizing device to the patient.

When in use to support a heel of a patient, for example, the pressure offloading device 100 may be positioned on the lower leg along the calf, above the prominence of the Achilles tendon and below the popliteal fossa such that it does not occlude the popliteal artery or vein. This may, for example, reduce the likelihood of irritation and may reduce or eliminate the creation of a pressure ulcer on the skin over the Achilles tendon or cutting off the blood supply to or return of blood from the lower leg to the heart. When in use, the leg is centered on the pressure offloading device 100, the sides of the pressure offloading device 100 are wrapped around the leg and secured by the strap, such that the strap does not come in contact with the shin of the leg. The pressure offloading device 100 extends up the calf to position the leg so that the foot is suspended in air and the knee is flexed and not hyperextended. For example, when the pressure offloading device 100 is in use, the knee of the patient may be flexed to a desired angle, e.g., at 5-10 degrees. The inner layer 102 of the pressure offloading device 100 conforms to the legs and allows for the leg to be rolled laterally without the pressure offloading device 100 coming off of the leg or shifting.

In some examples, the pressure offloading device may have portions that are partially or completely devoid of materials. The void of material in a portion of the pressure offloading device may be intentionally placed and fixed to the patient in order to support the area of a patient's body surrounding a particular area of concern (e.g., a donut-shaped pressure offloading device placed under the patient's abdomen to effectively offload a sacral ulcer with no portion of the device contacting the sacral ulcer or nearly surround area).

In some examples, the pressure offloading device 100 is configured to attach to a rigid u-shaped bar that loops around the foot with a soft material that holds the foot in the neutral position. In some examples, the pressure offloading device 100 is configured to attach to an essentially L-shaped bar that extends either over the toes or under the heel and engages the foot with a padded bar that supports the foot in a neutral position.

The respective layers may be formed from any suitable material that can provide a surface to support a portion of a person's body providing the desired level of firmness to sufficiently elevate or offset an area of the person's body while providing a sufficiently cushioned surface for comfort. While the embodiments in the Figures may be described with respect to foams, it will be appreciated that the layers in the pressure offloading device can be formed from other materials that provide the desired level of firmness to support a part of a patient's body and provide sufficient cushioning so as to be comfortable when wom by a patient. In one embodiment, the respective layers are independently formed from a foam material, a gel material, a thermoplastic elastomer, a rubber, a phase change material, or a combination of two or more thereof. The foam material can be selected from, for example, an open or closed cell foam. The foam can be either viscoelastic or non-viscoelastic. Suitable foams can include, without limitation, latex foams, natural latex foams, polyurethane foams, rubber foams, and the like. Examples of suitable foams include, but are not limited to, phenolic resin foams, polystyrene foams, polyurethane foams, polyethylene foams, polyvinylchloride foams, polyvinyl-acetate foams, polyester foams, polyether foams, and foam rubber. Examples of suitable gels include polyurethane gels, silicone gels, etc. Examples of thermoplastic elastomers include, but are not limited to, polyesters, elastomeric polyurethanes, elastomeric EVAs (ethylene/vinyl acetate copolymers), and others, such as silicone rubbers, polyurethanes and EP rubbers, e.g. EPDM rubbers. It will be appreciated that the respective layers within a device can be made from different types of materials. For example, different layers within a device could be made from different types of foams. There could be a layer formed from a foam and another layer formed from another material such as, for example, a gel or elastomeric material.

The softer, more compressive layers (i.e., those layers that may exhibit a lower deflection value and more prone to indentation) can also include other materials to provide a desired level of cushioning or softness. Such layers could include, for example, feathers, cotton stuffing, polymeric beads, etc. Additionally, such softer, high compression materials, could be provided over or around any of the layers described herein.

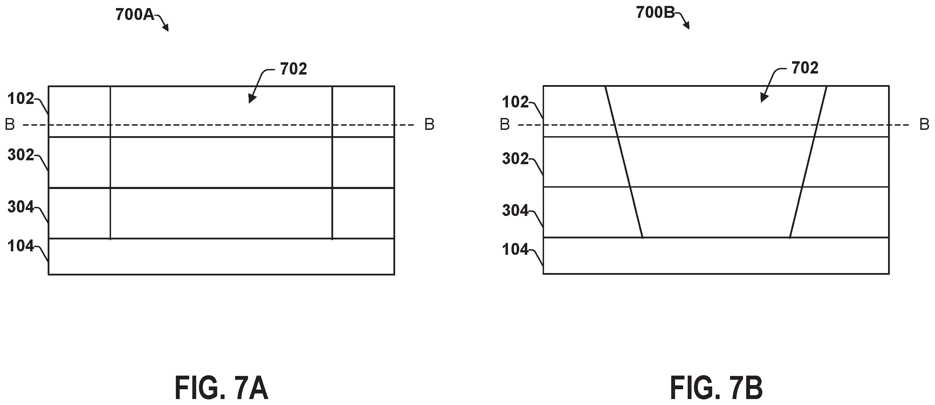

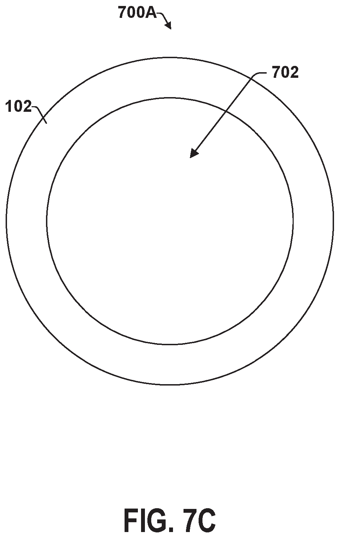

FIGS. 7A, 7B, and 7C illustrate example pressure offloading devices 700A and 700B configured to provide pressure reliefs for an area of the patient's body, such as the sacrum. In the illustrated examples, the pressure offloading devices 700A and 700B defines a cavity 702 for, for example, the tail bone or bony area to sit through. In the illustrated examples, the pressure offloading devices 700A and 700B includes the inner layer 102, the outer layer 104, the inner intermediate layer 302, and the outer intermediate area 304 as described above. In the illustrated example of FIG. 7A, the cavity 702 is cylindrical or elliptic cylindrical. In the illustrated example of FIG. 7B, the cavity 702 is conical or frustoconical. In the illustrated examples, the cavity 702 is defined by the inner layer 102, the inner intermediate layer 302, and the outer intermediate area 304, while the outer layer 104 provides a bottom to the cavity 702. Alternatively, in some examples, the cavity 702 may be defined by the inner layer 102, the inner intermediate layer 302, the outer intermediate area 304, and the outer layer 104. FIG. 7C illustrates a cross-sectional view of the pressure offloading devices 700A and 700B of FIGS. 7A and 7B from line B-B. In the illustrated example, the pressure offloading devices 700A and 700B have a circular cross-section. Alternatively, in some examples, the pressure offloading devices 700A and 700B may have an ovoid, rectangular, square, or any polygonal cross-section.

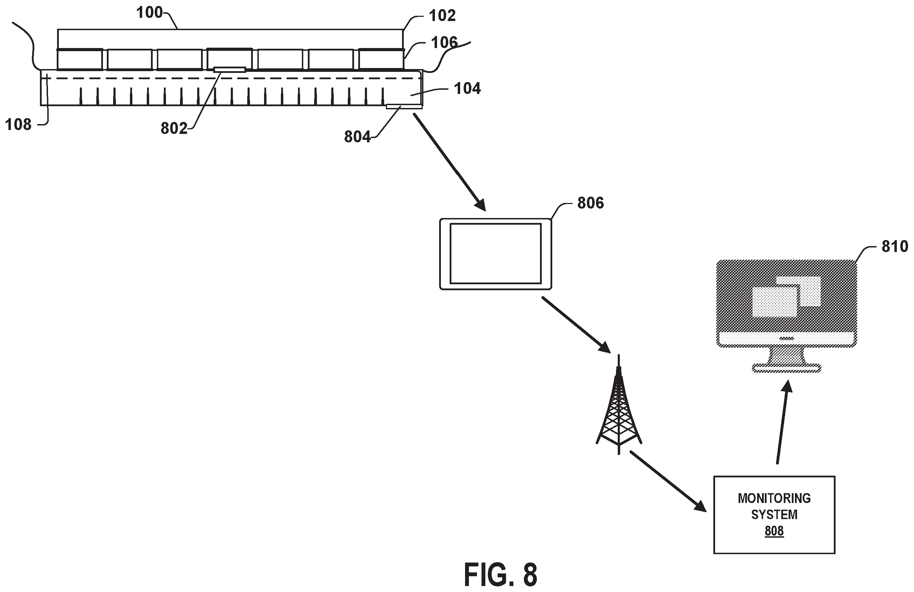

As illustrated in FIG. 8, the wound dressing, e.g., a heel dressing, may include one or more sensors 802 and a communication module 804. The sensor(s) 802 may include a sensor on the heel, a sensor that extends around the heel to the medial and lateral side of the ankle, or a plurality of sensors that extend around the heel to the medial and lateral side of the ankle. The sensors 802 are connected to a system, via the communication module 804, that alerts the patient or caregiver that the foot is no longer elevated. The sensors 802 may include (a) one or more gyroscopes that may indicate the patient's position, (b) one or more accelerometers to detect an angle and movement of the patient relative the pressure offloading device 100, (c) one or more pressure sensors and/or pressure sensor arrays to detect changes and/or shifts in pressure being applied to the pressure offloading device 100 that may indicative of the foot contacting the support surface, and/or (d) one or more temperature sensors to measure wound bed temperature, etc. The telemetry data collected by the sensors 802 may sometimes be referred to as "pose data."

In the illustrated example, the sensors 802 may be included in the pressure offloading device 100. In such an example, the sensors 802 may be embedded in and/or placed between foam layers 102, 104, 106 and 108 to protect the sensor 802 and/or to inhibit or prevent the sensor 802 from being noticed by the patient. In the illustrated example, the sensor 802 is embedded in the inner intermediate layer 106 and the outer intermediate layer 108 within one of the blocks 112. In some examples, the sensor 802 is positioned to be coaxial or in line with the expected force applied by the appendage or other body part when the pressure offloading device 100 is in use. The sensor 802 is communicatively coupled to the communication module 804, which is permanently or temporarily affixed to a side of the pressure offloading device 100 to reduce interference when the pressure offloading device 100 is attached to the patient.

The communication module 804 communicates to a computing device 506 (e.g., a stand-alone module, a smartphone, a smart watch, a tablet, a laptop computer, a work station, etc.) to produce alarms or otherwise notify the person or a caregiver when the heel is no longer suspended. In some examples, the communication module 804 includes a wireless control to wirelessly communicate (e.g., via Bluetooth^{®}, Bluetooth^{®} Low Energy (BLE), Digital Enhanced Cordless Telecommunications (DECT), any of the IEEE 802.11 standards, ZigBee^{®}, and/or Z-Wave^{®}, etc.) with the computing device 806. In some examples, the communication module 804 and/or the computing device 806 may be configured to forward sensor data to a monitoring system 808 that is geographically separated from pressure offloading device 100. The communication module 804 may be battery powered and may incorporate rechargeable batteries. In some examples where the batteries are not rechargeable, the sensors and communication module may be configured to have a battery life at least equal to the expected use time of the wound dressing, such as 10 days.

The monitoring system 808 may include an application operating on a workstation and a database that monitors one or more wound dressings. The monitoring system 808 may establish a fixed or user-adjustable threshold to indicate when contact is made over a period of time to eliminate false alarms. Additionally, in some examples, the monitoring system 808 detects when there is no sensor connected to, for example, the pressure offloading device 100 and provides a specific warning or indication to the caregiver or patient. The monitoring system 808 may include a database that enables the caregiver to observe the status of sensors on multiple people at one time, with detail as to the time and duration of contact, position of patient, and other information important to the patients' management. This database may reside locally in the institution or on the cloud.

The monitoring system 808 may communicate with an EMR application or an electronic health record (EHR) application running on one or more workstations 810 using communication and data protocols, such as HLA-7. The workstations 810 may be located at a central station (e.g., a nurse's station, etc.), may be located in the patient's room, and/or may be located remote from the care facility. The monitoring system 808 may communicate with a second database, transmitting identifiable or deidentified data to be aggregated for big data analysis. Data transmitted may include patient demographics, time, pressure and duration of contact, nutritional status, time to healing of the wound, and other pertinent data that would enable an investigator to understand healing of wounds across patients and centers and enable centers to compare their healing times with other centers in a confidential manner.

The pressure offloading device 100 may incorporate a sensing module (not shown) connected to at least one sensor on the dressing as described below, and a at least one light or audible system (not shown) such that it is self-contained with no communication other than the light or audible system. The light may be configured to glow one color when the foot is off-loaded and another color when the foot is not offloaded and requires attention.

While the above describes a pressure offloading device 100 and a monitoring system in terms of addressing a medical issue facing the heel, the disclosure devices and systems may be used to elevate and monitor other areas of the body, such as elbows, sacral, trochanter and ischial regions of the body.

The computing device 508 may directly or indirectly communicate with an application associated with a database (e.g., an electronic medical records (EMR) system) that enables a caregiver to observe the status of sensors on multiple people at one time, with detail as to the time and duration of contact, position of patient, and other information important to the patients' management. These databases may aggregate other information, such as nutrition status, blood electrolyte levels, blood pressure, and other demographic data such as wound staging and time to heel, to create benchmarks within and across institutions and develop best practice guidelines.

As described herein, the monitoring system, the wound dressing, and/or the pressure offloading device facilitate increased compliance when protecting a wound and protecting the wound from pressure.

Although the embodiments of this disclosure have been illustrated in the accompanying drawings and described in the foregoing detailed description, it is to be understood that the present disclosure is not to be limited to just the described embodiments, but that the embodiments described herein are capable of numerous rearrangements, modifications and substitutions without departing from the scope of the claims hereafter. It is, of course, not possible to describe every conceivable combination of components or methodologies for purposes of describing the present specification, but one of ordinary skill in the art may recognize that many further combinations and permutations of the present specification are possible. Each of the components described above may be combined or added together in any permutation to define an introducing device and/or introducing system. Accordingly, the present specification is intended to embrace all such alterations, modifications and variations that fall within the spirit and scope of the appended claims. Furthermore, to the extent that the term "includes" is used in either the detailed description or the claims, such term is intended to be inclusive in a manner similar to the term "comprising" as "comprising" is interpreted when employed as a transitional word in a claim. The claims as follows are intended to include all modifications and alterations insofar as they come within the scope of the claims or the equivalent thereof.
In view of the above, the present application discloses aspects and/or embodiments of the invention as described in the following itemized list:
1. A pressure offloading device configured to be secured to a portion of a person's body, the pressure offloading device comprising:
   an inner layer made of a first material having a first indentation force deflection (IFD) rating; and
   an outer layer made of a second material having a second IFD rating;
   wherein the second IFD rating is greater than the first IFD rating.
2. The pressure offloading device of item 1, wherein the first material and the second material are independently selected from a foam.
3. The pressure offloading device of item 2, further comprising a first intermediate layer between the inner layer and the outer layer.
4. The pressure offloading device of item 3, wherein the first intermediate layer comprises a third material selected from a foam with a third IFD rating.
5. The pressure offloading device of item 4, wherein the third IFD rating is greater than the first IFD rating and less than the second IFD rating.
6. The pressure offloading device of item 4, wherein the first intermediate layer comprises a first section of the third material, and a second section bordering the first section, the second section formed from a material other than the third material.
7. The pressure offloading device of item 6, wherein the material of the first section of the intermediate layer has an IFD rating greater than the first IFD rating, and the material of the second section of the intermediate layer has an IFD rating less than the IFD rating of the first section.
8. The pressure offloading device of item 3, wherein the inner layer has a first length and the first intermediate layer has a second length, and wherein the first length is longer than the second length.
9. The pressure offloading device of item 3, further comprising a second intermediate layer between the first intermediate layer and the outer layer, the second intermediate layer made of a fourth material selected from a foam having a fourth IFD rating.
10. The pressure offloading device of item 9, wherein the first intermediate layer is made of a third material selected from a foam with a third IFD rating, and wherein the fourth IFD rating is greater than the third IFD rating and less than the second IFD rating.
11. The pressure offloading device of item 9, wherein the inner layer includes a wing portion, wherein ends of the first and second intermediate layers are affixed to the wing portion.
12. The pressure offloading device of item 1, further comprising a first intermediate layer affixed to the inner layer and a second intermediate layer affixed to the first intermediate layer and the outer layer.
13. The pressure offloading device of item 1, further comprising:
   at least one intermediate layer between the inner layer and the outer layer,
   wherein the inner layer defines an inner side of the pressure offloading device,
   wherein each of the at least one intermediate layers has a higher IFD rating the further that layer is the from inner side of the pressure offloading device, and
   wherein the first IFD rating is the lowest IFD rating and the second IFD rating is the highest IFD rating.
14. The pressure offloading device of any of items 1-13, further comprising a cover, the cover having no seems at any point of contact between the cover and the portion of the person's body when the pressure offloading device is secured to the portion of the person's body.
15. The pressure offloading device of any of items 1-14, wherein the inner layer and the outer layer have a U-shape.
16. The pressure offloading device of item 15 wherein the outer layer includes a plurality of slits to facilitate the outer layer conforming to the U-shape.
17. A pressure offloading device comprising:
   multiple layers of foam affixed together having a U-shape with an inner side and an outer side, each of the multiple layers of foam having a different indentation force deflection (IFD) rating; and
   a cover with a fastener configured to secure the pressure offloading device to an appendage within the inner side.
18. The pressure offloading device of item 17, wherein the multiple layers of foam include:
   an inner layer on the inner side of the U-shape;
   a first intermediate layer affixed to the inner layer;
   a second intermediate layer affixed to the first intermediate layer; and
   an outer layer on the outer side of the U-shape affixed to the second intermediate layer.
19. The pressure offloading device of item 18, wherein:
   the IFD rating of the outer layer is greater than the IFD rating of the second intermediate layer, the IFD rating of the second intermediate layer is greater than the IFD rating of a center portion of the first intermediate layer, and
   the IFD rating of the center portion of the first intermediate layer is greater than the IFD rating of the inner layer.
20. The pressure offloading device of item 19, wherein:
   the IFD rating of the center portion of the first intermediate layer is at least 1.5 times greater than the IFD rating of the inner layer,
   the IFD rating of the second intermediate layer is at least 1.5 times greater than the IFD rating of a center portion of the first intermediate layer, and
   the IFD rating of the outer layer is at least 2 times greater than the IFD rating of the second intermediate layer.
21. The pressure offloading device of any of items 17-20, wherein the cover is configured to have no seems at any point of contact between the cover and the portion of the person's body when the pressure offloading device is secured to the appendage.
22. The pressure offloading device of any of items 17-20, wherein progression of the different IFD ratings of the multiple layers is configured to decrease pressure experienced by the appendage when the pressure offloading device is secured to the appendage.
23. A pressure offloading device configured to be secured to a portion of a person's body, the pressure offloading device comprising:
   a first layer including a single foam block having a first indentation force deflection (IFD) rating;
   a second layer connected to the first layer, the second layer including a central portion having a second IFD rating greater than the first IFD rating and edge portions having the first IFD rating;
   a third layer connected to the second layer, the third layer having a third IFD rating that is greater than the second IFD rating; and
   a fourth layer connected to the third layer, the fourth layer having a fourth IFD rating that is greater than the third IFD rating.
24. The pressure offloading device of item 23, further comprising a cover, the cover configured to have no seems at any point of contact between the cover and the portion of the person's body when the pressure offloading device is secured to the portion of the person's body.
25. The pressure offloading device of item 23 or 24, wherein the pressure offloading device has a U-shape when not secured to the portion of the person's body, and wherein the pressure offloading device wraps around the portion of the person's body when the pressure offloading device is secured to the portion of the person's body.
26. A method comprising:
   placing, in a U-Shaped cavity of a molding rig, an outer layer having a first indentation force deflection (IFD) rating;
   affixing, within the U-Shaped cavity of the molding rig, an outer intermediate layer onto the outer layer, the outer intermediate layer having a second IFD rating that is less than the first IFD rating;
   assembling an inner intermediate layer with a first potion having a third IFD rating and a second portion having a fourth IFD rating, the third IFD rating being less than the second IFD rating and the fourth IFD rating being less than the third IFD rating;
   affixing, within the U-Shaped cavity of the molding rig, the inner intermediate layer onto the outer intermediate layer; and
   affixing, within the U-Shaped cavity of the molding rig, an inner layer onto the inner intermediate layer, the inner layer having the fourth IFD rating.
27. A method of offloading an appendage of a patient, the method comprising:
   placing an appendage of a patient in the pressure offloading devices of any of items 1-25 to offload pressure from the appendage in contact with the pressure offloading device and/or an appendage distal from the offloading device.
28. The method of item 27 comprising securing, with a strap of the pressure offloading device, to the appendage to decrease pressure experienced by the appendage.

## Claims

1. A heel offloading device (100) configured to be secured to the lower leg of a patient , the heel offloading device (100) comprising:
an inner layer (102) made of a first material having a first indentation force deflection (IFD) rating;
an outer layer (104) made of a second material having a second IFD rating, wherein the second IFD rating is greater than the first IFD rating, wherein the inner and outer layers have a U-shape; and
a cover (600), the cover (600) having no seams (608) at any point of contact between the cover (600) and the lower leg when the heel offloading device is secured to the lower leg, wherein the heel offloading device (100) is configured to wrap around the lower leg when the heel offloading device (100) is secured to the lower leg.

2. The heel offloading device of claim 1, wherein the cover (600) is constructed in a U-shape conforming to the shape of the device.

3. The heel offloading device of claim 1 or 2, wherein the cover (600) comprises a strap (602) with a first fastening end (604) configured to fasten together with a second fastening end (606).

4. The heel offloading device of claim 3, being configured for the lower leg to be positioned in a location generally central to opposing ends of the heel offloading device, and the opposing ends of the heel offloading device are configured to be wrapped upwardly toward one another and secured using the strap (604).

5. The heel offloading device of any one of the preceding claims, further comprising one intermediate layer, wherein the IFD rating of a layer is 1.5 to 2.5 times the IFD rating of the adjacent layer that is closer to the inner layer (102).

6. The heel offloading device of any one of the preceding claims, wherein, as layers extend away from the lower leg, the layers are made of more resilient foams.

7. The heel offloading device of any one of the preceding claims, wherein the inner layer (102) comprises elongated portions (306) configured to connect to a corresponding inner edge (308) of the outer layer (104).
